# EUROPEAN PATENT APPLICATION

(11) **EP 4 682 241 A1**
(43) Date of publication of application: **21.01.2026**
(21) Application number: 24770891.0
(22) Date of filing: 12.03.2024
(51) Int. Cl.: C12N 1/04, C08L 51/08, C08L 71/02, C12N 5/00

(54) **CRYOPROTECTION OF CELLS**

(30) Priority: 13.03.2023 JP 2023039124
(71) Applicant: Celaid Therapeutics Inc., Tokyo 113-8485 (JP)
(72) Inventor: ISHIHARA Haruna, Tokyo 113-8485 (JP); ISHIDA Reiko, Tokyo 113-8485 (JP); KOHARA Hiroshi, Tokyo 113-8485 (JP); WATANABE Motoo, Tokyo 113-8485 (JP)
(74) Representative: Schön, Christoph
(86) International application number: PCT/JP2024/009601
(87) International publication number: WO 2024/190786

(57) **Abstract**

The present invention uses a composition for use in cell cryoprotection containing polyalkylene glycol modified with a copolymer containing a polyvinylcaprolactam block and a polyvinylacetate block. A method of producing cells may be utilized, wherein the method includes a freezing step of freezing a mixture comprising polyalkylene glycol modified with a copolymer containing a polyvinylcaprolactam block and a polyvinylacetate block and cells to generate frozen cells.

## Description

### TECHNICAL FIELD

The present invention relates to cryoprotection of cells.

Priority is claimed on Japanese Patent Application No. 2023-39124, filed March 13, 2023, the content of which is incorporated herein by reference.

### BACKGROUND ART

It is known that when cells are cryopreserved, the number of viable cells decreases after thawing. Therefore, the decrease in the number of viable cells has been suppressed by including a cryoprotectant in the composition containing the cells.

Dimethyl sulfoxide (DMSO) is often used as a cryoprotectant. On the other hand, the addition of hydroxyethyl starch (HES) and albumin to the composition has been reported to enhance the cryoprotective effect (e.g., Non Patent Documents 1 to 3).

### Citation List

### Non Patent Document

Non Patent Document 1: Stiff et al., Cryobiology. 1983 Feb;20(1):17-24.
Non-Patent Document 2: Stiff et al., Blood. 1987 Oct;70(4):974-8.
Non-Patent Document 3: Luo et al., Leuk Lymphoma. 1995 May;17(5-6):495-9.

### SUMMARY OF INVENTION

### Technical Problem

The cryoprotection method described above had room for improvement in terms of the inclusion of albumin that can cause biological contamination in the composition containing the cells. Solution to Problem

In the course of their research, the inventors investigated methods of cryoprotecting cells. As a result, the inventors were able to effectively cryoprotect cells without adding albumin by adding polyalkylene glycol modified with a copolymer containing a polyvinylcaprolactam block and a polyvinylacetate block in the composition. The inventors completed the invention based on this finding.

According to one aspect of the present invention, a composition for use in cell cryoprotection containing polyalkylene glycol modified with a copolymer containing a polyvinylcaprolactam block and a polyvinylacetate block is provided. This composition can be used to cryoprotect cells effectively.

According to one aspect of the present invention, a composition containing: polyalkylene glycol modified with a copolymer containing a polyvinylcaprolactam block and a polyvinylacetate block; and a cryoprotectant; is provided. This composition can be used to cryoprotect cells effectively.

According to one aspect of the invention, a method of producing cells, comprising a freezing step of freezing a mixture comprising polyalkylene glycol modified with a copolymer containing a polyvinylcaprolactam block and a polyvinylacetate block and cells to generate frozen cells, is provided. This method can be used to obtain cryoprotected cells effectively.

According to one aspect of the invention, a method of preserving cells, comprising a freezing step of freezing a composition containing polyalkylene glycol modified with a copolymer containing a polyvinylcaprolactam block and a polyvinylacetate block and cells to generate frozen cells, is provided. This method allows for effective cryoprotective preservation of cells.

According to one aspect of the invention, a method of freezing cells, comprising a freezing step of freezing a mixture comprising polyalkylene glycol modified with a copolymer containing a polyvinylcaprolactam block and a polyvinylacetate block and cells to generate frozen cells, is provided. This method can be used to cryoprotect cells effectively.

### Advantageous Effects of Invention

According to the present invention, cells can be effectively cryoprotected without the addition of albumin.

### BRIEF DESCRIPTION OF DRAWINGS

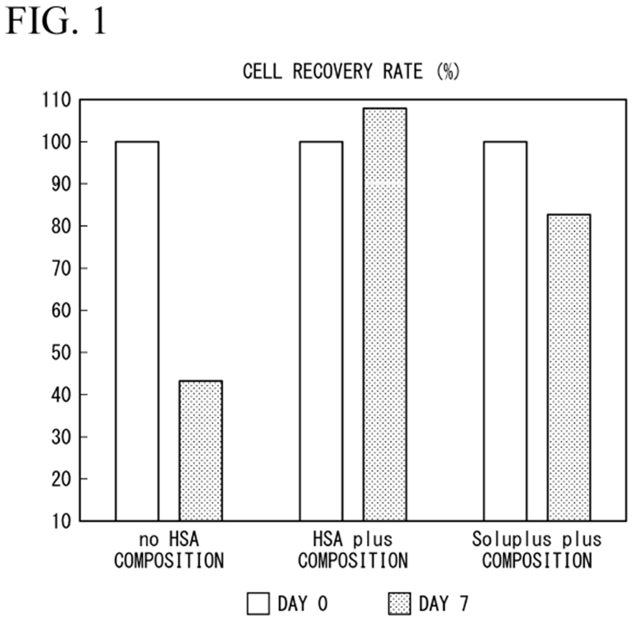

[FIG. 1] FIG. 1 shows the results of the recovery rate of cells after thawing.

### DESCRIPTION OF EMBODIMENTS

The following is a detailed description of the embodiment of the present invention. Similar contents will be omitted from the description as appropriate to avoid complications due to repetition.

### (1) Composition

According to one embodiment of the invention, a composition containing polyalkylene glycol modified with a copolymer containing a polyvinylcaprolactam block and a polyvinylacetate block is provided. Here, the composition may be a composition for addition for cell cryoprotection. The composition can be used to effectively cryoprotect cells. For example, the examples described in a later section show that the use of polyalkylene glycol modified with a copolymer containing a polyvinylcaprolactam block and a polyvinylacetate block as a cryoprotective component resulted in an improved recovery rate of cells after thawing. Further, according to one embodiment of the present invention, a method of freezing cells, a method of producing cells, a method of culturing cells, and a method of preserving cells using the composition, as well as a container containing the composition, are provided.

### (2) Composition

According to one embodiment of the present invention, a composition for use in cell cryoprotection, the composition containing polyalkylene glycol modified with a copolymer containing a polyvinylcaprolactam block and a polyvinylacetate block, is provided. This composition can be used to effectively cryoprotect cells. This composition may be a composition for addition.

### (3) Method

According to one embodiment of the present invention, a method is provided that comprises a step of freezing a mixture containing: polyalkylene glycol modified with a copolymer containing a polyvinylcaprolactam block and a polyvinylacetate block; and cells. This method can be used to effectively cryoprotect cells.

### (4) Freezing method

According to one embodiment of the present invention, a method of freezing cells, comprising a freezing step of freezing a mixture comprising polyalkylene glycol modified with a copolymer containing a polyvinylcaprolactam block and a polyvinylacetate block and cells to generate frozen cells, is provided. This method can be used to effectively cryoprotect cells. The method may comprise a generating step of generating a mixture comprising the polyalkylene glycol modified with a copolymer containing a polyvinylcaprolactam block and a polyvinylacetate block and cells, by contacting the polyalkylene glycol modified with a copolymer containing a polyvinylcaprolactam block and a polyvinylacetate block with a cell-containing composition.

### (5) Production method

According to one embodiment of the present invention, a method of producing cells, comprising a freezing step of freezing a mixture comprising polyalkylene glycol modified with a copolymer containing a polyvinylcaprolactam block and a polyvinylacetate block and cells to generate frozen cells, is provided. This method can be used to effectively obtain cryoprotected cells. The method may comprise a generating step of generating a mixture containing the polyalkylene glycol modified with a copolymer containing a polyvinylcaprolactam block and a polyvinylacetate block and cells, by contacting the polyalkylene glycol modified with a copolymer containing a polyvinylcaprolactam block and a polyvinylacetate block with a cell-containing composition.

### (6) Preserving method

According to one embodiment of the present invention, a method of preserving cells, comprising a freezing step of freezing a mixture comprising: polyalkylene glycol modified with a copolymer containing a polyvinylcaprolactam block and a polyvinylacetate block and cells, to generate frozen cells, is provided. This method can be used to effectively preserve cells in a cryoprotected state. The method may comprise a generating step of generating a mixture comprising polyalkylene glycol modified with a copolymer containing a polyvinylcaprolactam block and a polyvinylacetate block, and cells, by contacting: the polyalkylene glycol modified with a copolymer containing a polyvinylcaprolactam block and a polyvinylacetate block with a cell-containing composition.

### (7) Culture method

According to one embodiment of the present invention, a method of culturing cells, comprising: a freezing step of freezing a mixture comprising polyalkylene glycol modified with a copolymer containing a polyvinylcaprolactam block and a polyvinylacetate block and cells to generate frozen cells; a thawing step of thawing said frozen cells to generate thawed cells; and a culturing step of culturing the thawed cells, is provided. This method can be used to effectively obtain cryoprotected cells. The method may comprise a generating step of generating a mixture comprising polyalkylene glycol modified with a copolymer containing a polyvinylcaprolactam block and a polyvinylacetate block and cells, by contacting the polyalkylene glycol modified with a copolymer containing a polyvinylcaprolactam block and a polyvinylacetate block with a cell-containing composition.

### (8) Preserving method

According to one embodiment of the present invention, a method of preserving cells, comprising a freezing step of freezing a composition containing polyalkylene glycol modified with a copolymer containing a polyvinylcaprolactam block and a polyvinylacetate block and cells, is provided. This method can be used to effectively preserve cells in a cryoprotected state. The composition may contain a cryoprotectant.

### (9) Preserving method

According to one embodiment of the present invention, a method of preserving cells, comprising a step of placing a composition containing polyalkylene glycol modified with a copolymer containing a polyvinylcaprolactam block and a polyvinylacetate block and cells in a freezing environment, is provided. This method can be used to effectively preserve cells in a cryoprotected state. The composition may comprise a cryoprotectant.

### (10) Assisting Method

According to one embodiment of the present invention, a method of assisting cryoprotection, comprising a step of generating a mixture by mixing polyalkylene glycol modified with a copolymer containing a polyvinylcaprolactam block and a polyvinylacetate block, a cryoprotectant, and cells, is provided. This method can be used to assist or enhance the cryoprotective effect of the cryoprotectant on the cells.

### (11) Composition

According to one embodiment of the present invention, a composition for assisting in cryoprotection, containing polyalkylene glycol modified with a copolymer containing a polyvinylcaprolactam block and a polyvinylacetate block, for adding to a composition containing a cryoprotectant or cells, is provided. The composition can be used to assist or enhance the cryoprotective action of the cryoprotectant.

### (12) Enhancement method

According to one embodiment of the present invention, a method of enhancing cryoprotection, comprising a step of generating a mixture by mixing polyalkylene glycol modified with a copolymer containing a polyvinylcaprolactam block and a polyvinylacetate block and a cryoprotectant, is provided. This method can be used to enhance the cryoprotection of cells by the cryoprotectant.

### (13) Improvement method

According to one embodiment of the present invention, a method for improving the cryoprotection of cells, comprising a step of generating a mixture by mixing polyalkylene glycol modified with a copolymer containing a polyvinylcaprolactam block and a polyvinylacetate block, a cryoprotectant, and cells, is provided. This method can be used to effectively cryoprotect cells.

### (14) Production method

According to one embodiment of the present invention, a method of producing a composition comprising a step of mixing polyalkylene glycol modified with a copolymer containing a polyvinylcaprolactam block and a polyvinylacetate block and a cell-containing composition, is provided. The composition obtained by this method can be used to effectively cryoprotect cells.

### (15) Method of use

According to one embodiment of the present invention, a method of use of a composition containing polyalkylene glycol modified with a copolymer containing a polyvinylcaprolactam block and a polyvinylacetate block for use in cell cryoprotection, is provided. According to this method of use, cells can be effectively cryoprotected. This method of use may be carried out to generate a mixture compsiring polyalkylene glycol modified with a copolymer containing a polyvinylcaprolactam block and a polyvinylacetate block and a cell-containing composition.

### (16) Composition

According to one embodiment of the present invention, a composition containing 0.001% (w/v) or more polyalkylene glycol modified with a copolymer containing a polyvinylcaprolactam block and a polyvinylacetate block, is provided. This composition can be used to effectively cryoprotect cells.

### (17) Container

According to one embodiment of the present invention, a container containing a composition containing polyalkylene glycol modified with a copolymer containing a polyvinylcaprolactam block and a polyvinylacetate block. The container can be used to effectively cryoprotect cells, is provided. The container may further contain cells or a cryoprotectant. The container may be filled with polyalkylene glycol modified with a copolymer containing a polyvinylcaprolactam block and a polyvinylacetate block and cells and optionally a cryoprotectant, and the container may be frozen.

### (18) Cell

According to one embodiment of the present invention, cells or cell population thereof is obtained by each of the methods described in (3) to (10) or (12) to (15) above, or (21) below. The cells or cell population thereof are inhibited from damage caused by freezing. Therefore, the obtained cells or cell population can be effectively used for cell transplantation or cell culture.

### (19) Container

According to one embodiment of the present invention, a container is obtained that contains cells or cell population thereof obtained by each of the methods described in (3) to (10) or (12) to (15) above or (21) below. The cells or cell population thereof contained in this container are inhibited from damage caused by freezing.

### (20) Pharmaceutical composition

According to one embodiment of the present invention, a pharmaceutical composition or method of producing thereof, the pharmaceutical composition containing the cells or cell population thereof obtained by each of the methods described in (3) to (10) or (12) to (15) above or (21) below,is provided.

### (21) Transplantation method

According to one embodiment of the present invention, a method of transplantation is provided. This transplantation method may include, for example, a step of transplanting the cells or cell population thereof or a pharmaceutical composition containing such cells or cell population obtained by each of the methods described in (3) to (10) or (12) to (15) above, into a subject (e.g., human or mammal other than human). According to this transplantation method, the disease can be treated by effective cell engraftment. This transplantation method may include each of the methods described in each of (3) to (10) or (12) to (15) above.

### (22) Composition

According to one embodiment of the present invention, a composition for use in any of the methods described in (3) to (10), (12) to (15) or (21) above, the composition containing polyalkylene glycol modified with a copolymer containing a polyvinylcaprolactam block and a polyvinylacetate block, a cryoprotectant, or cells, is provided.

### (23) Frozen product

According to one embodiment of the present invention, a frozen product of the composition according to (1), (2), (11), (16), (20) or (22) above is provided. The frozen product has a superior cell recovery rate when thawed.

### (24) Step

In one embodiment of the present invention (e.g., including (3) to (10), (12) to (15) or (21) above) the method may comprise one or more of the following (i) to (iii): (i) a generating step of generating a mixture comprising polyalkylene glycol modified with a copolymer containing a polyvinylcaprolactam block and a polyvinylacetate block and cells, by contacting the polyalkylene glycol modified with a copolymer containing a polyvinylcaprolactam block and a polyvinylacetate block with a cell-containing composition.; (ii) a freezing step of freezing a mixture containing polyalkylene glycol modified with a copolymer containing a polyvinylcaprolactam block and a polyvinylacetate block and cells, to generate frozen cells, or (iii) a thawing step of thawing the frozen cells to generate thawed cells. Also, in one embodiment of the present invention (e.g. including (3) to (10), (12) to (15) or (21) above), the method may comprise any one or more of the following (iv) to (vi): (iv) a step of generating a mixture by mixing polyalkylene glycol modified with a copolymer containing a polyvinylcaprolactam block and a polyvinylacetate block and a cryoprotectant; (v) a step of generating a mixture of polyalkylene glycol modified with a copolymer containing a polyvinylcaprolactam block and a polyvinylacetate block, a cryoprotectant and cells by contacting a mixture containing the polyalkylene glycol modified with a copolymer containing a polyvinylcaprolactam block and a polyvinylacetate block and the cryoprotectant with a cell-containing composition; and (vi) a freezing step of freezing a mixture containing polyalkylene glycol modified with a copolymer containing a polyvinylcaprolactam block and a polyvinylacetate block, cells and a cryoprotectant to generate frozen cells. Also, in one embodiment of the present invention (e.g., including (3) to (10), (12) to (15) or (21) above), the method may comprise one or more of the following (vii) to (ix): (vii) a step of generating a composition containing cultured cells by culturing cells, (viii) a step of collecting the composition containing the cultured cells (e.g., a step of obtaining a precipitate fraction by separating the composition containing the cultured cells into a supernatant and a precipitate fraction by centrifugation), or (ix) a step of generating a mixture comprising containing polyalkylene glycol modified with a copolymer containing a polyvinylcaprolactam block and a polyvinylacetate block by contacting a solution containing polyalkylene glycol modified with a copolymer containing a polyvinylcaprolactam block and a polyvinylacetate block and the precipitate fraction containing cells. Also, in one embodiment of the present invention (e.g. including (3) to (10), (12) to (15) or (21) above), the method may comprise one or more steps of the following (x) to (xvi). (x) a step of generating a mixture by contacting a cell-free aqueous solution containing polyalkylene glycol modified with a copolymer containing a polyvinylcaprolactam block and a polyvinylacetate block with a cell-containing composition and optionally a cryoprotectant; (xi) a freezing step of freezing the mixture containing polyalkylene glycol modified with a copolymer containing a polyvinylcaprolactam block and a polyvinylacetate block and the cells and optionally the cryoprotectant to generate frozen cells; (xii) a step of placing a container containing the frozen cells under non-freezing conditions (thawing conditions); (xiii) a step of transplanting the cells after thawing into a living organism; (xiv) a step of culturing the cells after thawing; (xv) a step of collecting the cultured cells, or (xvi) a step of transferring the cultured cells to a container. Employing one or more of these steps is useful for effective cryoprotection of cells. When two or more steps are employed from embodiments of the present invention (including, e.g., the method of (3) to (10), (12) to (15) or (21) above, or the step of (i) to (xvi) above), the order is arbitrary, and the order can be determined according to the desired operation. Each method or step of the embodiments of the present invention (including, e.g., the method of (3) to (10), (12) to (15) or (21), above, or the step of (i) to (xvi)) may be performed in vitro or ex vivo.

In an embodiment of the present invention (including e.g., (1) to (24) above), effective cryoprotection includes a superior recovery rate of viable cells after freeze-thawing the cells. The recovery rate is preferably greater than 70%, as the FDA guidance (see Guidance for FDA Reviewers and Sponsors. Content and Review of Chemistry, Manufacturing, and Control (CMC) Information for Human Somatic Cell Therapy Investigational New Drug Applications (INDs). U.S. Department of Health and Human Services Food and Drug Administration Center for Biologics Evaluation and Research. April 2008: 1-39., and Guidance for Industry. Biologics License Applications for Minimally Manipulated, Unrelated Allogeneic Placental/Umbilical Cord Blood Intended for Hematopoietic and Immunologic Reconstitution in Patients with Disorders Affecting the Hematopoietic System. U.S. Department of Health and Human Services Food and Drug Administration Center for Biologics Evaluation and Research. March 2014: 1-49), recommends the cryoprotection process with a survival rate of 70% or higher. Recovery rates may be 70, 75, 80, 85, 90, 95, or 100%, and may be within any two of those values. Superior or improved recovery rate includes improved recovery rate compared to conditions without polyalkylene glycol modified with a copolymer containing a polyvinylcaprolactam block and a polyvinylacetate block. The recovery rate may be expressed as (number of viable cells in the medium after freezing and thawing / number of viable cells in the same medium before freezing) x 100 (%). The recovery rate may be expressed as (the number of viable cells in a container after freezing and thawing / the number of viable cells in the same container before freezing) × 100 (%).

In an embodiment of the present invention (including e.g., (1) to (24) above), the concentration of polyalkylene glycol modified with a copolymer containing a polyvinylcaprolactam block and a polyvinylacetate block in a composition containing cells and the polyalkylene glycol modified with a copolymer containing a polyvinylcaprolactam block and a polyvinylacetate block (e.g., a pre-freezing composition prepared for cryopreservation, a composition immediately before freezing, or a composition during freezing), may be, for example, 0.0001%, 0.0005%, 0.001%, 0.002%, 0.003%, 0.004%, 0.005%, 0.006%, 0.007%, 0.008%, 0.009%, 0.01%, 0.015%, 0.02%, 0.03%, 0.05%, 0.1%, 0.2%, 0.5%, 1%, 2%, or 5% (w/v) or higher, and may fall within a range between any two of the above values. The concentration may be, for example, 0.001% to 0.1% (w/v), 0.002% to 0.05% (w/v), 0.002% to 0.01% (w/v), 0.003% to 0.01% (w/v), 0.003% to 0.008% (w/v), or 0.004% to 0.006% (w/v).

In an embodiment of the present invention (e.g., including (1) to (24) above), the concentration of a polyalkylene glycol modified with a copolymer containing a polyvinylcaprolactam block and a polyvinylacetate block in a composition containing the polyalkylene glycol modified with a copolymer containing a polyvinylcaprolactam block and a polyvinylacetate block (e.g., a composition prior to mixing with cells, a composition for addition to be mixed with cells, or a composition for addition to be mixed with a cryoprotectant) may be, for example, 0.0002%, 0.001%, 0.002%, 0.004%, 0.006%, 0.008%, 0.01%, 0.012%, 0.014%, 0.016%, 0.018%, 0.02%, 0.03%, 0.04%, 0.06%, 0.1%, 0.2%, 0.4%, 1%, 2%, 4%, or 10% (w/v) or higher, and may fall within a range between any two of the above values. The concentration may be, for example, 0.002% to 0.2% (w/v), 0.004% to 0.1% (w/v), 0.004% to 0.02% (w/v), 0.006% to 0.02% (w/v), 0.006% to 0.016% (w/v), or 0.008% to 0.012% (w/v). The composition containing the polyalkylene glycol modified with a copolymer containing a polyvinylcaprolactam block and a polyvinylacetate block may further contain a cryoprotectant. The composition containing the polyalkylene glycol modified with a copolymer containing a polyvinylcaprolactam block and a polyvinylacetate block may be mixed with a cell-containing composition for the purpose of cryoprotection of the cells. In this case, the cell-containing composition may be mixed with the composition in an amount of, for example, 0.5-, 0.6-, 0.7-, 0.8-, 0.9-, 1-, 1.1-, 1.2-, 1.3-, 1.4-, 1.5-, or 2-fold relative to the amount of the composition.

In an embodiment of the present invention (e.g. including (1) to (24) above), the polyalkylene glycol modified with a copolymer containing a polyvinylcaprolactam block and a polyvinylacetate block may be, for example, polyethylene glycol modified with a copolymer containing a polyvinylcaprolactam block and a polyvinylacetate block. The polyalkylene glycol modified with a copolymer containing a polyvinylcaprolactam block and a polyvinylacetate block may be, for example, polyvinylcaprolactam-polyvinylacetate-polyethylene glycol graft copolymer. The polyalkylene glycol modified with a copolymer containing a polyvinylcaprolactam block and a polyvinylacetate block may be, for example, graft copolymers derived from (i) N-vinylcaprolactam, (ii) vinylacetate, and (iii) polyether. The polyether may be polyethylene glycol. The polyalkylene glycol modified with a copolymer containing a polyvinylcaprolactam block and a polyvinylacetate block may be, for example, a compound obtained by free radical polymerization of a mixture of (i) to (iii) above. For example, (i) may be 40 to 60 wt%, (ii) may be 15 to 35 wt%, and (iii) may be 10 to 30 wt%. (i) may be, for example, 40, 45, 50, 55, 57 or 60 wt%, and may be in the range of any two of those values. (ii) may be, for example, 15, 20, 25, 30, or 35 wt%, and may be within any two of those values. (iii) may be, for example, 10, 13, 15, 20, 25, or 30 wt% and may be within any two of those values. Above (i) to (iii) may be 100 wt% in total. The polyalkylene glycol modified with a copolymer containing a polyvinylcaprolactam block and a polyvinylacetate block may be, for example, compound of the following structural formula (1) or salts thereof.

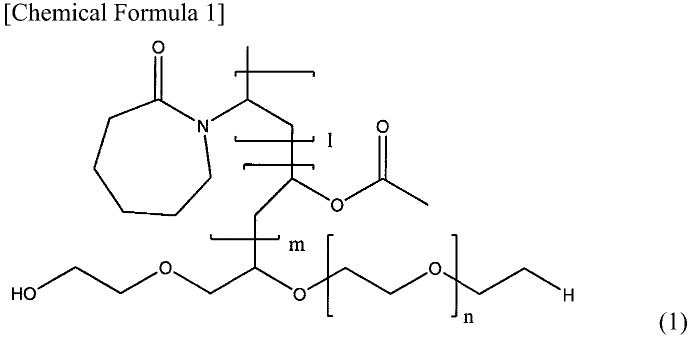

Here, the wt% of 1, m, and n may be, for example, 40 to 60 wt%, 15 to 35 wt%, and 10 to 30 wt%, respectively. The wt% of 1 may be, for example, 40, 45, 50, 55, 57, or 60, and may be within any two of those values. The wt% of m may be, for example, 15, 20, 25, 30, or 35, and may be within any two of those values. The wt% of n may be, for example, 10, 13, 15, 20, 25, or 30, and may be within any two of those values. The wt% of 1, m, n above may be 100 wt% in total. The wt% of 1, m and n may be, for example, 56 to 58 wt%, 29 to 31 wt%, and 12 to 14 wt%, respectively, and may be about 57, about 30, and about 13 wt%, respectively. The polymerization degrees of 1, m, and n may be, for example 60 to 160, 470 to 1110, and 480 to 1130, respectively. The degree of polymerization of 1 may be, for example, 60, 70, 80, 90, 100, 110, 120, 130, 140, 150 or 160, and may be within any two of those values. The degree of polymerization of m may be, for example, 470, 500, 550, 600, 650, 700, 750, 780, 800, 850, 900, 950, 1000, 1050 or 1110 and may be within any two of those values. The degree of polymerization of n may be, for example, 480, 500, 550, 600, 650, 700, 750, 780, 800, 850, 900, 950, 1000, 1050 or or 1130, and may be in the range of any two of those values. The degree of polymerization may be the average degree of polymerization. The mass average molecular weight (Mw) of the polyalkylene glycol modified with a copolymer containing a polyvinylcaprolactam block and a polyvinylacetate block may be, for example, 70000 g/mol or higher. This value may be, for example, 70000, 80000, 90000, 100000, 120000, 140000, 150000, 160000, or 170000 g/mol, and may be in the range of any two of those values. This value may be, for example, 70000 to 170000 g/mol, 80000 to 160000 g/mol, 90000 to 140000 g/mol, 100000 to 130000 g/mol, 110000 to 125000 g/mol, or 117000 to 119000 g/mol. This value may be about 118000 g/mol. The polyalkylene glycol modified with a copolymer containing a polyvinylcaprolactam block and a polyvinylacetate block may be, for example, Soluplus. Soluplus is a compound having the structure of formula (1) above. The value 1, m, and n of Soluplus may have the above wt% or degrees of polymerization. For the method of producing polyalkylene glycol modified with a copolymer containing a polyvinylcaprolactam block and a polyvinylacetate block, for example, the method described in US 2008/0293828 A1, US 2010/0204425 A1 or US 2018/0305636 A1 may be employed. For the components of (i) to (iii) above, the components described in these references may also be used.

In an embodiment of the present invention (including, for example, (1) to (24) above), the freezing includes placing the composition or container under freezing conditions. The freezing conditions include, for example, placing the composition or container below freezing point. The freezing conditions may be, for example, 0, -10, -20, -40, -60, -80, -100, -120, -140, -150, -160, or - 180°C or lower, and may be within any two of those values. The freezing time may be, for example, 0.25, 0.5, 0.75, 1, 1.5, 2, 3, 4, 5, 6, 7, 10, 50, or 100 days or more, and may be within any two of those values. The freezing may be performed by placing the subject composition or container in liquid nitrogen or in a freezer. The compositions subject to freezing include compositions containing cells, polyalkylene glycol modified with a copolymer containing a polyvinylcaprolactam block and a polyvinylacetate block, or a cryoprotectant. The containers subject to freezing include containers containing the composition. The non-freezing environment includes, for example, placing the composition or container in an environment above 0°C (e.g., 37°C).

In an embodiment of the present invention (including, for example, (1) to (24) above), the cryoprotection includes preventing damage to cells that may occur when placed under freezing conditions. The damage includes damage resulting from freezing of water during freezing. The damage includes damage to cell membranes caused by pressure resulting from freezing of water.

In an embodiment of the present invention (e.g., including (1) to (24) above), cryoprotectants include, for example, DMSO, HES, glycerol, trehalose, or sorbitol. The cryoprotectants can be produced by known methods. The commercially available cryoprotectant can be used, and may be purchased from manufacturers (e.g., Kyokuto Pharmaceutical Industries, Ltd., Fujifilm Wako Pure Chemicals Corporation, Tokyo Kasei Kogyo Co., etc.). The concentration of the cryoprotectant (e.g., if the composition has more than one cryoprotectants, the concentration of each cryoprotectant (e.g., containing DMSO or HES, etc.)) in a composition containing cells and a cryoprotectant (including a composition before freezing that is prepared for cryopreservation, a composition just before freezing, or a composition during freezing)may be, for example, 1, 2, 3, 4, 5, 6, 7, 8, 9, 10, 11, 12, 13, 14, 15, or 20% (w/v or v/v) or more, and may be within any of those two values. The concentration of the cryoprotectant in a composition containing a polyalkylene glycol modified with a copolymer containing a polyvinylcaprolactam block and a polyvinylacetate block and a cryoprotectant (e.g., including a composition prior to mixing with cells or a composition for addition to be mixed with cells) (if the composition has more than one cryoprotectants, the concentration of each cryoprotectant(e.g., containing DMSO or HES, etc.)) may be, for example, 2, 3, 4, 5, 6, 7, 8, 9, 10, 11, 12, 13, 14, 15, 20 or 40% (w/v or v/v) or more, or within any of those two values.

In an embodiment of the present invention (including, for example, (1) to (24) above), thawing may be carried out by transferring cells from frozen material under freezing conditions to non-freezing conditions. The thawing may be carried out, for example, by bringing the container containing the frozen material into contact with a water bath at about 37°C.

In an embodiment of the present invention (including, for example, (1) to (24) above), addition involves adding an additional substance to the composition. The addition may be carried out, for example, to generate a mixture comprising polyalkylene glycol modified with a copolymer containing a polyvinylcaprolactam block and a polyvinylacetate block and a cell-containing composition. This effectively cryoprotects the cells. Addition may also be carried out, for example, to generate a mixture comprising the polyalkylene glycol modified with a copolymer containing a polyvinylcaprolactam block and a polyvinylacetate block and the composition containing the cryoprotectant. This may enhance the cryoprotective effect of the composition containing the cryoprotectant.

In an embodiment of the present invention (including, for example, (1) to (24) above), the composition may be in the form of, for example, a solution, a medium, or an additive. The composition may contain, for example, polyalkylene glycol modified with a copolymer containing a polyvinylcaprolactam block and a polyvinylacetate block, cells, or a cryoprotectant. The composition may be, for example, albumin-free. Free includes a state in which the target component is not added to the composition, a state in which the target component is entirely absent from the composition, or a state in which the composition does not contain the component in concentrations above the detection limit. Albumin-free includes being substantially albumin-free. The additive may be an additive to be mixed into the cell-containing composition to enhance the cryoprotective effect of the cells. The term additive may be used interchangeably with a composition for addition. The embodiments of compositions can be applied to mixtures as well.

In an embodiment of the present invention (including, for example, (1) to (24) above), a frozen product may be generated by placing the composition under freezing conditions. The frozen product includes the frozen form of the composition. The frozen product is preferably albumin-free.

In an embodiment of the present invention (including, for example, (1) to (24) above) the cells may be mammalian cells. Mammals include, for example, humans, monkeys, rodents (mice, hamsters, etc.), rabbits, dogs, cats, horses, cattle, sheep, pigs, goats, marmosets, etc. Cells include, for example, somatic cells or stem cells. Somatic cells include, for example, cells derived from skin, heart, liver, lung, stomach, intestine, kidney, uterus, brain, blood, or mesenchymal tissue. Cells also include, for example, CD34+ cells, immune cells (e.g., T cells), cells for regenerative medicine, CHO cells, or malignant tumor cells. Stem cells include, for example, pluripotent, bipotent, or monopotent stem cells. Pluripotent stem cells include, for example, iPS cells or ES cells. Bipotent stem cells include, for example, mesenchymal stem cells, adipose stem cells, hematopoietic stem cells, and neural stem cells. Unipotent stem cells include, for example, muscle stem cells, pigment stem cells, etc. Hematopoietic stem cells can be harvested from umbilical cord, bone marrow, placenta, and peripheral blood. Human hematopoietic stem cells include CD34+ cells. These cells may be purchased from a manufacturing or marketing company (e.g., StemExpress or Lonza). CD34+ cells or hematopoietic stem cells may be obtained by fractionating CD34+ cells from cord blood, peripheral blood, or bone marrow. Cells include viable cells. Cells may be present in the form of cell populations. A cell population includes, for example, a plurality of cells resulting from cell division. The percentage of any of the cells listed above (e.g., CD34+ cells) in the cell population may be, for example, 10, 20, 40, 60, 80, or 100%, and may be within any two of those values. The percentage of any of the cells listed above (e.g., CD34+ cells) to the total cells in a medium or container may be, for example, 10, 20, 40, 60, 80, or 100%, and may be within any two of those values.

In an embodiment of the present invention (including, for example, (1) to (24) above), the medium includes the medium used for culturing cells. The base component of the medium may be any common base component and there is no compositional limitations. The base components of the medium may include, for example, amino acids, peptides, proteins, inorganic salts, vitamins, minerals, or carbon sources (e.g., glucose). Base components of the medium may include, for example, GlutaMAX, L-alanine L-glutamine dipeptide, L-glutamine, ITS-X, insulin, transferrin (apo), sodium selenite, ethanolamine, or Flt3 ligand. The medium may include, for example, a basic medium for cell culture. Basic media may include, for example, S-clone SF-3 medium, F12 medium, StemSpan (Stem Cell technologies), STEM Alpha (STEM ALPHA), StemPro-34 serum-free medium (Gibco Invitrogen), StemPro MSC serum-free medium (Invitorogen), HSC-CFU medium (Miltenyl Biotech), S-Clone serum-free medium (SF-02, SF-03, CM-B, SF-B) (Sanko Junyaku), HPGM medium (Sanko Junyaku), AIM V medium (Invitorogen), Marrow MAX bone marrow medium (Invitrogen), KnockOut DMEM/F-12 medium (Invitrogen), Stemline hematopoietic stem cell expansion medium (Sigma), SYN serum-free media (SYN H, SYN B) (AbCys SA), SPE IV medium (AbCys SA), MyeloCult medium (StemCell Technologies), HPG serum-free medium (Lonza), UltraCULTURE medium (Lonza), Opti-MEM medium (Gibco Invitrogen etc.), MEM medium (Gibco Invitrogen etc.), MEMα (Gibco Invitrogen etc.), DMEM medium (Gibco Invitrogen etc.), IMDM medium (Wako Pure Chemical etc.), PRMI1640 medium (Gibco Invitrogen etc.), Ham F-12 medium (Gibco etc.), or RD medium. The medium is preferably albumin (e.g., GenBank Accession No. AAN17825.1) -free or serum-free. In this case, it is possible to suppress instability in maintaining undifferentiated characteristics and issues of biological contamination. The medium may further contain Soluplus (registered trademark) (CAS Registry Number 402932-23-4). The medium may also contain an antibiotic (e.g., penicillin or streptomycin). The medium may contain hematopoietic stem cells. The medium may be in a liquid or solid form. Culturing cells using the medium includes culturing the cells in the medium.

In an embodiment of the present invention (including, for example, (1) to (24) above), culturing includes incubating cells under conditions suitable for growth or maintenance. The culturing may include a step of generating a cell-containing composition by contacting the medium and the cells and a step of incubating the cell-containing composition. The incubating step may be performed with the cell-containing composition standing or stirring. The incubation may be performed at about 37°C and under a 5% CO₂ atmosphere. The culturing time of the cells may be, for example, 0.5, 1, 2, 3, 4, 5, 6, 8, 10, 12, 14, 16, 17, 18, 20, 30 or 35 days or longer, and may be within any two of those values. This culturing time may be, for example, 1 to 20 days, 3 to 17 days, 4 to 16 days, or 5 to 14 days. The culturing includes the incubation time of the medium containing the cells.

In an embodiment of the present invention (e.g. including (1) to (24) above), the cell density in the composition containing cells at the time of freezing may be, for example, 1×10³, 1×10⁴, 1×10⁵, 2×10⁵, 3×10⁵, 4×10⁵, 5×10⁵, 6×10⁵, 8×10⁵, 1×10⁶, 5×10⁶, 1×10⁷, 1×10⁸, or 1×10⁹ cells/mL or higher, and may be in the range of any two of those values. This density may be, for example, 1×10³ to 1×10⁸, 1×10⁴ to 1×10⁷, 1×10⁴ to 5×10⁶, or 1×10⁵ to 5×10⁶ cells/mL.

In an embodiment of the present invention (including, for example, (1) to (24) above), in a composition containing cells and polyalkylene glycol modified with a copolymer containing a polyvinylcaprolactam block and a polyvinylacetate block, or in a composition containing cells, polyalkylene glycol modified with a copolymer containing a polyvinylcaprolactam block and a polyvinylacetate block and a cryoprotectant, the cell density in the composition may be, for example, 1×10³, 1×10⁴, 1×10⁵, 2×10⁵ , 3×10⁵, 4×10⁵, 5×10⁵, 6×10⁵, 8×10⁵, 1×10⁶, 5×10⁶, 1×10⁷, 1×10⁸, or 1×10⁹ cells/mL or higher, and may be in the range of any two of those values. This density may be, for example, 1×10³ to 1×10⁸, 1×10⁴ to 1×10⁷, 1×10⁴ to 5×10⁶, or 1×10⁵ to 5×10⁶ cells/mL.

In an embodiment of the present invention (e.g. including (1) to (24) above), contacting may be carried out by mixing a composition containing a substance with another composition containing another substance. The contacting of cells and polyalkylene glycol modified with a copolymer containing a polyvinylcaprolactam block and a polyvinylacetate block may be carried out by mixing a cell suspension with a solution containing polyalkylene glycol modified with a copolymer containing a polyvinylcaprolactam block and a polyvinylacetate block. The contacting of cells and polyalkylene glycol modified with a copolymer containing a polyvinylcaprolactam block and a polyvinylacetate block may be carried out by mixing a cell fraction (including e.g. pellets) with a solution containing polyalkylene glycol modified with a copolymer containing a polyvinylcaprolactam block and a polyvinylacetate block. The contacting of cells, polyalkylene glycol modified with a copolymer containing a polyvinylcaprolactam block and a polyvinylacetate block and a cryoprotectant may be carried out by mixing the cell suspension or cell fraction with a solution containing polyalkylene glycol modified with a copolymer containing a polyvinylcaprolactam block and a polyvinylacetate block and a cryoprotectant.

In an embodiment of the present invention (including, for example, (1) to (24) above), the cell-containing composition may be in the form of a cell suspension or cell fraction. The cell fraction includes, for example, cell pellets.

In an embodiment of the present invention (e.g. including (1) to (24) above), collection may comprise one or more of the following steps: step of obtaining a precipitate fraction by separating a composition containing medium and cultured cells into a supernatant and a precipitate fraction by a centrifugation process; a step of generating a cell suspension by suspending the precipitate fraction containing the cells in an aqueous solution; a step of transferring desired cells from a container containing cells to another container; or a step of fractionating or purifying the desired cells . The collection may be performed in a sterile environment.

In an embodiment of the present invention (including, for example, (1) to (24) above), the preservation includes cryopreservation. The preservation includes, for example, placing cells, a composition, or a container containing cells or composition in a frozen environment. The preservation period may be, for example, 0.25, 0.5, 0.75, 1, 1.5, 2, 3, 4, 5, 6, 7, 10, 50, or 100 days or longer, or may be within any two of those values.

In an embodiment of the present invention (including, for example, (1) to (24) above), containers include tubes, vials, ampules, or bottles. The containers include containers for freezing. The containers for freezing include containers that are resistant to breakage due to freezing. The container may contain a composition containing polyalkylene glycol modified with a copolymer containing a polyvinylcaprolactam block and a polyvinylacetate block, cells, or a cryoprotectant.

In an embodiment of the present invention (including, for example, (1) to (24) above), "+" includes the finding that the cells are positive for the molecule identified by the term immediately preceding it.

Any publication cited herein is hereby incorporated by reference in its entirety. In the present specification, "or" is used when "at least one or more" of the items listed in the text can be employed. The same applies to "or". Whenever "within a range of two values" is specified herein, the range includes the two values themselves. In the present specification, "A to B" includes A and B. In the present specification, "(1) to (24) above" includes references to any one or more of (1), (2), (3), (4), (5), (6), (7), (8), (9), (10), (11), (12), (13), (14), (15), (16), (17), (18), (19), (20), (21), (22), (23) or ( 24).

The above-described embodiments of the invention are examples of forms that can be included in the present invention. The present invention is not limited to these embodiments, and various configurations other than the above can be employed. The present invention may also employ each of the configurations or features described in the above embodiments in combination or independently.

### Examples

The invention is not limited to these examples, which are further described below.

### 1. cryopreservation test

### 1.1 Method

Three compositions shown in Table 1 were prepared. CP-1 (registered trademark) High Grade (hereafter CP-1) (Kyokuto Pharmaceutical Industries, Ltd.) was used as the solution containing dimethyl sulfoxide (DMSO) and hydroxyethyl starch (HES) In the first composition, human serum albumin (HSA) was not added to CP-1 (No. 1, no HAS Composition). In the second composition, HSA was added to CP-1 according to the instructions for CP-1 (No. 2, HSA plus composition). In the third composition, Soluplus (registered trademark)(polyalkylene glycol modified with a copolymer containing a polyvinylcaprolactam block and a polyvinylacetate block) was added to CP-1 (No. 3, Soluplus plus composition).

**[Table 1]**

| Table 1. Composition | | | | |
|---|---|---|---|---|
| No. | Name of Composition | Components | Final Concentration (in physiological saline) | Note |
| 1 | no HSA Composition | DMSO | 10%(v/v) | |
| | | HES | 12%(w/v) | |
| | | HSA | 0% | |
| 2 | HSA plus Composition | DMSO | 10%(v/v) | |
| | | HES | 12%(w/v) | |
| | | HSA | 6.4%(w/v) | 20% donated albumin |
| 3 | Soluplus plus Composition | DMSO | 10%(v/v) | |
| | | HES | 12%(w/v) | |
| | | HSA | 0% | |
| | | Soluplus | 0.01%(w/v) | 10% Stock |

5 µM 740Y-P, 0.1 µM butyzamide, 1 µM UM729, and 0.01% Soluplus (registered trademark) were added to IMDM (Iscove's Modified Dulbecco's Media) containing 1xGlutaMAX, 1xITS-X, and 10ng/ml Flt3- ligand. Human cord blood CD34+ cells thawed according to the manufacturer's instructions were suspended in the resulting medium and plated in 96-well plates. The medium was exchanged every 3 to 4 days. On day 10 of culture, all CD34+ cell progeny in the wells were collected in a 1.5 mL tube (Eppendorf tube). The tubes were centrifuged and the supernatant was removed. The precipitate was suspended in saline to obtain a cell suspension. The cell suspension was placed in a cryotube and an equal volume of each of the compositions in Table 1 (No. 1, 2 or 3) was added slowly. The cryotubes containing the resulting mixture were stored overnight in a freezer at -80°C . The next day, the cryotubes were transferred to liquid nitrogen; after 7 days, cells were thawed using a water bath and viable cells were measured.

The materials used in this experiment are listed below.

**[Table 2]**

| Table 2. Reagents | | | |
|---|---|---|---|
| Name | Catalog Number | Vendor | Lot No. |
| IMDM | 098-06465 | Wako Pure Chemical (Osaka, Japan) | DLF7009 |
| GlutaMAX (100x) | 35050-061 | Gibco (CA, USA) | #2270532 |
| ITS -X | 51500-056 | Gibco (CA, USA) | #2415083 |
| UM729 | 72332 | Stemcell Technologies (Vancouver, Canada) | 1000064161 |
| 740Y-P | N/A | Scrum (Tokyo, Japan) | SQ25426 |
| Soluplus | N/A (Free sample) | BASF (Ludwigshafen, Germany) | N/A |
| Butyzamide | N/A | Shionogi Pharmaceutical (Osaka, Japan) | N/A |
| Flt3-ligand | AF-300-19 | Peprotech (NJ, USA) | |
| CP-1 High Grade | 27207 | Kyokuto Pharmaceutical Industrial (Tokyo, Japan) | |
| Physiological saline | - | Otsuka Pharmaceutical (Tokyo, Japan) | |

| | | | |
|---|---|---|---|
| N/A: Not applicable | | | |

**[Table 3]**

| Name | Catalog Number | Vendor | Lot No. |
|---|---|---|---|
| Human umbilical cord blood CD34⁺ cells | CBP3400.5C | StemExpress (CA, USA) | |
| Corning CellBIND 96-well plate | 3300 | Corning Inc. (NY, USA) | |

### 1.2 Results

Results are shown in Figure 1. When the mixture of cell suspension and no HSA composition was frozen, the recovery rate of cells after thawing was as low as about 43%. When the mixture of cell suspension and HSA plus composition was frozen, the recovery rate of cells after thawing was as high as about 108%. When the mixture of cell suspension and Soluplus plus composition was frozen, the recovery rate of cells after thawing was as high as about 83%. These results indicate that the addition of Soluplus improves the recovery rate of cells after thawing.

In the past, the use of albumin as a cryoprotective component has been attempted, but albumin is a molecule that can cause biological contamination. Albumin also has the problem of high raw material cost. On the other hand, this experiment showed that Soluplus can be used as a cryoprotective component instead of albumin. Soluplus can be chemically synthesized and is less expensive than albumin. Furthermore, when used as a cryoprotective component, the amount of Soluplus added can be kept to a small amount. Therefore, the use of Soluplus is superior in terms of safety and convenience.

The present invention has been described above on the basis of examples. It is understood by those skilled in the art that the examples are illustrative only, that various variations are possible, and that such variations are also within the scope of the invention.

### INDUSTRIAL APPLICABILITY

According to the invention, polyalkylene glycol modified with a copolymer containing a polyvinylcaprolactam block and a polyvinylacetate block can be added to the composition to effectively cryoprotect cells without the addition of albumin, making the invention industrially applicable.

## Claims

1. A composition for use in cell cryoprotection containing polyalkylene glycol modified with a copolymer containing a polyvinylcaprolactam block and a polyvinylacetate block.

2. The composition according to claim 1, wherein the polyalkylene glycol modified with a copolymer containing a polyvinylcaprolactam block and a polyvinylacetate block is the compound represented by the following structural formula (1). 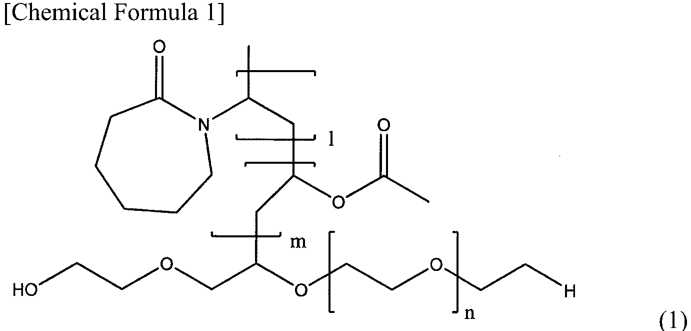 wherein wt% of 1, m, and n are 40 to 60 wt%, 15 to 35 wt%, and 10 to 30 wt%, respectively.

3. The composition according to claim 1 or 2, wherein said composition is a composition for addition and said addition is performed to produce a mixture comprising: polyalkylene glycol modified with a copolymer containing a polyvinylcaprolactam block and a polyvinylacetate block; and a cell-containing composition.

4. The composition according to claim 1 or 2, wherein said composition is a composition for addition and said addition is performed to produce a mixture comprising: polyalkylene glycol modified with a copolymer containing a polyvinylcaprolactam block and a polyvinylacetate block; a cell-containing composition; and a cryoprotectant.

5. The composition according to claim 1 or 2, further containing a cryoprotectant.

6. The composition according to claim 1 or 2, containing more than 0.001% (w/v) of polyalkylene glycol modified with a copolymer containing a polyvinylcaprolactam block and a polyvinylacetate block.

7. A composition containing: polyalkylene glycol modified with a copolymer containing a polyvinylcaprolactam block and a polyvinylacetate block and a cryoprotectant.

8. The composition according to claim 7, containing 0.001% (w/v) or more polyalkylene glycol modified with a copolymer containing a polyvinylcaprolactam block and a polyvinylacetate block.

9. The composition according to claim 7 or 8, which is albumin-free.

10. A method of producing cells, comprising:
freezing step of freezing a mixture comprising: polyalkylene glycol modified with a copolymer containing a polyvinylcaprolactam block and a polyvinylacetate block; and cells, to generate frozen cells.

11. The method of producing cells according to claim 10, comprising:
generating step of generating a mixture comprising: polyalkylene glycol modified with a copolymer containing a polyvinylcaprolactam block and a polyvinylacetate block; and cells, by contacting the polyalkylene glycol modified with a copolymer containing a polyvinylcaprolactam block and a polyvinylacetate block with a cell-containing composition.

12. The method according to claim 11, wherein said generating step includes generating a mixture comprising: the polyalkylene glycol modified with a copolymer containing a polyvinylcaprolactam block and a polyvinylacetate block; a cryoprotectant; and the cells, by contacting: the polyalkylene glycol modified with a copolymer containing a polyvinylcaprolactam block and a polyvinylacetate block; the cryoprotectant; and the cell-containing composition.

13. A method of preserving cells comprising:
freezing step of freezing a composition containing a polyalkylene glycol modified with a copolymer containing a polyvinylcaprolactam block and a polyvinylacetate block and cells, to generate frozen cells.

14. The method according to claim 13, wherein said composition contains 0.001% (w/v) or more polyalkylene glycol modified with a copolymer containing a polyvinylcaprolactam block and a polyvinylacetate block.

15. The method according to claim 13 or 14, wherein said composition contains a cryoprotectant.

16. A method of freezing cells comprising:
generating step of generating a mixture comprising polyalkylene glycol modified with a copolymer containing a polyvinylcaprolactam block and a polyvinylacetate block and cells, by contacting the polyalkylene glycol modified with a copolymer containing a polyvinylcaprolactam block and a polyvinylacetate block with a cell-containing composition; and
freezing step of freezing the mixture to generate frozen cells.

17. The method according to claim 16, wherein said generating step includes generating a mixture comprising the polyalkylene glycol modified with a copolymer containing a polyvinylcaprolactam block and a polyvinylacetate block, a cryoprotectant, and the cells, by contacting: the polyalkylene glycol modified with a copolymer containing a polyvinylcaprolactam block and a polyvinylacetate block; the cryoprotectant; and the cell-containing composition.
